# EUROPEAN PATENT SPECIFICATION

(11) **EP 1 742 620 B1**
(45) Date of publication and mention of the grant of the patent: **05.12.2012**
(21) Application number: 05713582.4
(22) Date of filing: 10.02.2005
(51) Int. Cl.: A61K 9/70, A61K 47/34, A61K 31/00, C08L 83/04, A61L 15/58, A61K 47/10

(54) **METHOD OF MAKING SILICONE PRESSURE SENSITIVE ADHESIVES FOR DELIVERING HYDROPHILIC DRUGS USING A SILICONE POLYETHER**
VERFAHREN ZUR HERSTELLUNG VON SILIKON-KONTAKTKLEBERN ZUR ABGABE VON HYDROPHILEN ARZNEIMITTELN UNTER VERWENDUNG EINES SILIKONPOLYETHERS
PROCEDE DE FABRICATION D'ADHESIFS SENSIBLES A LA PRESSION EN SILICONE POUR ADMINISTRER DES MEDICAMENTS HYDROPHILES A L'AIDE D'UN POLYETHER DE SILICONE

(30) Priority: 12.03.2004 US 553036 P
(43) Date of publication of application: 17.01.2007
(73) Proprietor: Dow Corning Corporation, Midland, MI 48686-0994 (US)
(72) Inventor: RAUL, Victor, Albert, Midland, MI 48640 (US); SCHALAU, Gerald, K. II, Freeland, MI 48623 (US); THOMAS, Xavier, B-1640 Rhode-Saint-Genese (BE)
(74) Representative: Gillard, Richard Edward
(86) International application number: PCT/US2005/004758
(87) International publication number: WO 2005/092300

(56) References cited:
- US-A- 4 814 184
- US-A- 4 840 796
- US-A- 5 035 894
- US-A- 5 114 707
- US-A- 5 556 636
- US-A- 5 658 975
- US-A1- 2003 180 281
- DOW CORNING: "Product Information: Dow Corning 9011"[Online] 26 April 2002 (2002-04-26), XP002330255 Retrieved from the Internet: URL:http://www.dowcorning.com/DataFiles/09 0007b580108c93.pdf> [retrieved on 2005-06-01]

## Description

This invention relates to methods of making hydrophobic matrices containing silicone pressure sensitive adhesives and solid powdered hydrophilic drugs and/or solid powdered hydrophilic excipients. More particularly, the improvement according to the invention resides in pre-forming a semi-solid composition containing the solid powdered hydrophilic drug and/or the solid powdered hydrophilic excipient, and a silicone polyether. The pre-formed semi-solid composition is then added to a silicone pressure sensitive adhesive or to a solution containing a solvent and a silicone pressure sensitive adhesive.

Silicone adhesive compositions can be pressure sensitive adhesives or permanent bonding type adhesives. Permanent bonding implies that the adhesive will actually cement two surfaces together, i.e., it behaves like a glue. Pressure sensitive, on the other hand, means that the adhesive can be stripped from a surface and re-adhered to a surface, i.e., it has the nature of the adhesive present on SCOTCH® brand tapes. While the focus of this invention is the pressure sensitive type of adhesive, the same components used herein can be used to create permanent bonding type adhesives with such components, if desired. Thus, in order to prepare an adhesive which will provide a permanent bond, it is required that a suitable crosslinking agent such as a hydrogen bearing silicone polymer and a catalyst be included along with the components of the pressure sensitive adhesive.

Typically, a silicone pressure sensitive adhesive comprises (i) a silicone resin containing monofunctional (M) units R₃SiO_{1/2} and tetrafunctional (Q) units SiO₄, i.e., an MQ silicone resin, wherein R is a hydrocarbon group, optionally a hydrocarbon group having 1-20 carbon atoms such as methyl, ethyl, propy, hexenyl, phenyl and the like; and (ii) a polydiorganosiloxane fluid or gum, optionally a high molecular weight hydroxyl endblocked polydiorganosiloxane fluid with a viscosity of 100 to 1,000,000, alternatively 5,000 to 1,000,000 centistokes (mm²/s) or a high molecular weight hydroxyl endblocked polydiorganosiloxane gum where viscosity is expressed in terms of plasticity. Other ingredients know for use in silicone pressure sensitive adhesives can also be incorporated.

Silicone pressure sensitive adhesives can be prepared by simply mixing components (i), (ii) and any other optional pressure sensitive adhesive ingredients. Generally, this takes place in the presence of a mutual solvent such an organic, aromatic, hydrocarbon or silicone solvent, i. e. , ethyl acetate, heptane, xylene, or toluene. However, the solvent can be omitted. As soon as the components are mixed, the composition is ready for use as a pressure sensitive adhesive without further treatment. It can simply be applied to the surfaces to be adhered by any suitable means, and then the surfaces are brought together. Typically, if the composition contains a solvent, the solvent is allowed to evaporate before adhering the two surfaces. The coating can be cured for a short time by heating it briefly, although curing is not generally required. Likewise, a catalyst can be added to assist in the curing, although a catalyst is not generally required.

While the focus of this invention is primarily directed to silicone pressure sensitive adhesives of the type described in US Patent 4, 655, 767 (April 7,1987), the'767 patent, which is considered incorporated herein by reference, other types of silicone pressure sensitive adhesives can be used, if desired. Thus, other types of silicone pressure sensitive adhesives which can be used are described, for example, in US Patent 2,736, 721 (February 28,1956) ; US Patent 2,814, 601 (November 26,1957) ; US Patent 2,857, 356 (October 21,1958) ; US Patent 4,584, 355 (April 22,1986) ; US Patent 4,585, 836 (April 29,1986) ; US Patent 4,591, 622 (May 27,1986) ; and US Patent 5,482, 988 (January 9,196), the'988 patent; all of which are considered incorporated herein by reference. In addition, other types of adhesives having a more suitable surface pressure sensitive adhesion property can be used, such as the so-called Soft Skin Adhesive, i.e., the siloxane gel compositions described in detail in US Patent 5,145, 933 (September 8,1992), which are prepared from (A) alkenyl-containing polydiorganosiloxanes, (B) hydrosilicon compounds having at least three SiH groups, (C) SiH end-blocked polydiorganosiloxanes, and a (D) catalyst.

None of these references, however, either describe or suggest the method of making silicone pressure sensitive adhesive compositions according to this invention. In addition, and with particular regard to the'988 patent, the compositions prepared according to the method described in the present invention exhibit a greater resistance to deformation than the compositions in the'988 patent which possess a much lower resistance to deformation. Furthermore, while it's possible in some instances, to use the waxy type silicone polyethers of the'988 patent, in the method according to this invention, the benefits derived and attributed to the method of the present invention would be compromised.
US 5,035,894 discloses adhesive compositions comprising blends of polyethylene oxide-grafted silicone polymers with resinous copolymers. US 5,658,975 discloses hot-melt silicone pressure sensitive adhesive compositions with siloxylated polyether waxes as additives. US 4,840,796 discloses transdermal drug delivery systems comprising, among other essential ingredients, a matrix containing a medicinally active ingredient. US 5,556,636 discloses sticky compositions for medical use comprising a tackifier resin and an oxyalkylene polymer having a silicon-containing group. US 5,114,707 discloses polycondensation silicone elastomer dosage forms adapted for the continuous and controlled release of iodine, particularly to domestic water supplies. US application 2003/018028 discloses topical preparations comprising a hydrophilic carrier and an active agent dispersed in a silicone matrix. US 4,814,184 discloses pharmaceutical delivery devices comprising a hydrophilic or polar pharmaceutically acceptable material dispersed in a silicone matrix.

It is known that hydrophilic drugs and/or hydrophilic excipients are not soluble in hydrophobic matrices of silicone pressure sensitive adhesives. It is also known that the addition of hydrophilic materials to silicone pressure sensitive adhesives, generally results in the formation of large crystals and/or agglomerates, and that the crystals and agglomerates cannot be evenly distributed in the matrices of silicone pressure sensitive adhesives. This results in products containing low levels of drug and/or excipient, or products containing varying and inconsistent quantities of drugs and/or excipients.

However, and in accordance with the present invention, by preparing a slurry of the hydrophilic drug and/or excipient in a silicone polyether, and then adding the pre-prepared slurry of the hydrophilic drug and/or hydrophilic excipient to a silicone pressure sensitive adhesive or to a solvated silicone pressure sensitive adhesive, the hydrophilic drug and/or the hydrophilic excipient become stable in their soluble form, or are present in the hydrophobic matrix of the silicone pressure sensitive adhesive in very small discrete particles.

Among the benefits achieved according to this invention are products containing the silicone pressure sensitive adhesive and the hydrophilic drugs and/or hydrophilic excipients possess improved physical stability and an improved rate of drug release. The presence of the silicone polyether also results in additional tack-adhesion properties which increase wear properties of transdermal patches containing the hydrophobic silicone pressure sensitive adhesive matrix. Cohesiveness of the silicone pressure sensitive adhesive is not compromised, and the silicone polyether enables skilled artisans to successfully include hydrophilic materials into the hydrophobic matrices of silicone pressure sensitive adhesives.

In particular, the present invention is directed to a method of making a hydrophobic adhesive matrix, for example one containing a silicone pressure sensitive adhesive, and a solid powdered hydrophilic drug or a solid powdered hydrophilic excipient. The steps of the method consist of (i) the formation of a semi-solid composition, i.e., slurry, containing a solid powdered hydrophilic drug or a solid powdered hydrophilic excipient, and a silicone polyether. In the second step, a silicone pressure sensitive adhesive or a solution containing a solvent and a silicone pressure sensitive adhesive are combined with the semi-solid composition. The semi-solid composition and the silicone pressure sensitive adhesive or the solution containing the solvent and the silicone pressure sensitive adhesive are mixed together to form a hydrophobic matrix. The hydrophobic matrix can then be applied to a substrate, typically human skin by means of a transdermal patch for the continuous and controlled transdermal administration of drugs.

Generally, the ratio of the solid powdered hydrophilic drug and/or the solid powdered hydrophilic excipient to the silicone polyether in the semi-solid composition is not critical. It can, for example, be in a ratio of 1:100 to 100:1, alternatively 1:10 to 10:1, and alternatively 1:1 weight ratio. When a solution of silicone pressure sensitive in a solvent is used, it typically contains 10-90 percent by weight of the silicone pressure sensitive adhesive and 10-90 percent by weight of the solvent, alternatively 30-80 percent by weight of the silicone pressure sensitive adhesive and 20-70 percent by weight of the solvent.

These and other features of the invention will become apparent from a consideration of the detailed description.

### DESCRIPTION

The term *drug* as used herein is intended to mean substances defined as drugs under the Federal Food, Drug, and Cosmetic Act, Pub. L. No. 75-717, 52 STAT. 1040 (1938), 21 USC Sec. 201. [321]. Generally, drugs according to Sec. 201 [321] (g)(1) (B) and (C) are substances intended for use in the diagnosis, cure, mitigation, treatment, or prevention of disease in man or other animals; and substances, other than food, intended to affect the structure or any function of the of the body of man or other animal.

Some representative examples of such substances are (i) drugs that act upon the central nervous system such as clozapine, risperidone, chordiazepoxide, buspirone, desipramine, maprotiline, amitriptyline, timolol, selegiline, naloxone and nalbuphine; (ii) drugs affecting renal and cardiovascular function such as acetazolamide, isosorbide, furosemide, chlorothiazide, amiloride, captopril, enalapril, lisinopril, isosorbide nitrate, nifedipine, verapamil, phenytoin, lidocaine, propranolol, amiodarone, pravastatin, probucol and ciprofibrate; (iii) drugs affecting gastrointestinal function such as cimetidine, omeprazole and ranitidine; (iv) drugs for the treatment of helminthiasis such as thiabendazole and mebendazole; (v) drugs for the treatment of microbial diseases such as trimethoprim, norfloxacin, ciprofloxacin, penicillin G nafcillin, cephalothin cefazolin, kanamycin A, neomycin, doxycycline minocycline, clarithromycin, clindamycin, flucytosine, ketoconazole, fluconazole, acyclovir and ganciclovir; (vi) drugs for the treatment of neoplastic diseases such as dacarbazine, busulfan, and triazenes; (vii) drugs for the treatment of nutrient deficiency such as folic acid, niacinamide, ascorbic acid and thiamine; (viii) drugs for hormonal replacement therapy such as estradiol, ethinyl estradiol and norethindrone; (ix) drugs that inhibit the synthesis and actions of adrenocortical hormones such as cortisol, cortisone and prednisone; and (x) drugs used in dermatology for the treatment of dermatoses such as betamethasone dipropionate, hydrocortisone, dexamethasone sodium phosphate, retinal, tretinoin, isotretinoin, dapsone, calipotriene, ketoconazole, clotrimazole, itraconazole and arotinoid.

The term *excipients* as used herein is intended to mean substances as defined in the Handbook of Pharmaceutical Excipients, Ray. C. Rowe, Paul J. Weller, and Arthur H. Kibbe, (Editors), as additives used to convert pharmacologically active compounds into pharmaceutical dosage forms suitable for the administration to patients. Some representative examples of such additives are (i) sugars and sugar derivatives such as acacia, dextrin, dextrose, fructose, lactose, maltodextrin, mannitol, sorbitol, sucrose, and xylitol; (ii) starch derivatives; (iii) cellulosic materials such as sodium carboxymethylcellulose, microcrystalline cellulose, cellulose acetate phthalate, sodium croscarmellose, methyl cellulose, ethylcellulose, hydroxyethylcellulose, hydroxypropylcellulose, hydroxypropylmethylcellulose, and hydroxypropylmethylcellulose phthalate; (iv) polysaccharides such as dextrates, guar gum, and xanthan gum; (v) polyethers such as poloxamer and polyoxyethylene alkyl ethers; (vi) polyvinyl alcohols; (vii) acrylic and methacrylic acid polymers such as Carbopol, Carbomer, polacrilin potassium, and polymethacrylates; (viii) pyrrolidone derivatives such as povidone and crospovidone; (ix) glycuronam polymers and derivatives such as alginic acid and the calcium and sodium alignate salts thereof; (x) solid diluents such as the calcium and magnesium salts of carbonates, calcium phosphate derivatives, calcium sulfate, magnesium oxide, potassium chloride, and potassium citrate; (xi) solid lubricants such as calcium and magnesium stearate derivatives, talc, and zinc oxide; (xii) suspending agents such as kaolin, magnesium aluminum silicate, carbon, and cyclodextrins; and (xiii) others excipient substances such as cholesterol, fumaric acid, lecithin, gelatin, malic acid, sodium bicarbonate, sodium citrate salts, sodium stearyl fumarate, titanium dioxide, and zinc oxide.

While nearly any silicone polyether can be used, the silicone polyethers are preferably copolymeric silicone polyethers containing dimethylsiloxy units and oxyalkylene units in their molecule. These materials often have a degree of polymerization (DP) generally less than about twenty. Such silicone polyethers are well know in the art, commercially available, and described in detail in detail in US Patent 3,402,192 (September 17, 1968), and more recently in, for example, US Patent 6,121,373 (September 19, 2000). If desired, other types of silicone polyethers can be used, but it may result in failure to obtain all of benefits of the invention.

Thus, some representative examples of other types of silicone polyethers which may be considered can be found described in detail in the following Patents, (i) crosslinked silicone polyethers in US Patent 5,136,068 (August 4, 1992); (ii) waxy silicone polyethers in US 5,482,988 (January 9, 1996); (iii) oligomeric silicone polyethers in US Patent 5,488,124 (January 30, 1996); (iv) short chain low molecular weight silicone polyethers and cyclic silicone polyethers in US Patent 5,623,017 (April 22, 1997); (v) oxyalkylene functional silanes in US Patent 5,707,550 (January 13, 1998); (vi) elastomeric silicone polyethers in US Patent 5,811,487 (September 22, 1998); and (vii) silicone polyethers containing arylalkyl groups in US Patent 6,133,370 (October 17, 2000); all of which are considered incorporated herein by reference thereto.

In the context of the present invention, the term *slurry* is intended to mean a semi-solid composition containing a solid powdered drug and/or a solid powdered excipient, and a silicone polyether. The components of the semi-solid composition are generally present in a weight ratio of 1:100 to 100:1, alternatively 1:10 to 10:1, and alternatively 1:1, i.e., one part of the silicone polyether and one part of the drug, excipient, or drug and excipient.

The pressure sensitive adhesives used in the invention are described above. Some mutual and compatible solvents for the pressure sensitive adhesives which can be used in the method according to the invention include organic, aromatic and hydrocarbon solvents such as ethyl acetate, heptane, benzene, xylene, or toluene. Silicone fluids can also be used as solvent including low molecular weight short chain linear siloxanes such as hexamethyldisiloxane, octamethyltrisiloxane, and decamethyltetrasiloxane, and cyclic siloxanes such as octamethylcyclotetrasiloxane (D₄) and decamethylcylocpentasiloxane (D₅). These compositions can be used in the dilution/solvation of silicone pressure sensitive adhesives. The use of a solvent is optional, however, and a solvent can be omitted in those instances where solventless silicone pressure sensitive adhesives are desired. This is common practice, for example, in the customization of solventless silicone pressure sensitive adhesives having adjustable tack.

The method of the present invention consists of first making a slurry, i.e., a semi-solid composition, of the solid powdered hydrophilic drug and/or the solid powdered hydrophilic excipient, and a silicone polyether. This slurrying process allows any agglomerations of the solid powdered hydrophilic drugs to be broken up into solutions/finely dispersed particles, which in turn prevents their random and uncontrolled crystallization. It also facilitates incorporation of such materials into other liquid silicones, i.e., liquids into liquids by matching liquid viscosity. Typically, the slurrying process provides a lower shear system, and therefore the drug stability is not significantly affected. Additionally, the use of silicone polyethers as the surfactant enables their participation in the release kinetics of the blend. Thus, it is know that hydrophilic materials such as silicone polyethers can function to increase release kinetic profiles by (i) homogeneously dispersing the hydrophilic phase, i.e., the excipient phase, and by (ii) stabilizing hydrophilic phases into hydrophobic silicone containing phases, with the result that the hydrophilic behavior or property of the blend is increased.

In the second step, the pre-formed semi-solid composition is mixed with a silicone pressure sensitive adhesive or a solution containing a solvent and the silicone pressure sensitive adhesive to form the hydrophobic matrix. As noted before, the process of this invention will function with nearly any adhesive matrix, alternatively any hydrophobic adhesive matrix.

The hydrophobic matrix can be applied to suitable backing materials or substrates by any conventional means such as roller coating, dip coating, extrusion, knife coating, or spray coating. No special equipment is needed to carry out the method, and simple laboratory mortars and pestles can be employed, as well as air-driven or electric general purpose mixers.

### EXAMPLES

The following examples are set forth in order to illustrate the invention in more detail.

The silicone polyether used in these examples was a copolymeric silicone polyether containing dimethylsiloxy units and oxyalkylene units. It had a degree of polymerization (DP) of about fifteen, and its structure generally corresponded to the structure of the silicone polyether of Formula (I) in US Patent 6,121,373 (September 19, 2000), wherein the sum of x and y were 15.

The silicone pressure sensitive adhesive used in the examples was composed of (i) an MQ resin, and (ii) a hydroxyl endblocked polydiorganosiloxane fluid having a degree of polymerization (DP), i.e., the number of repeat units, of about 1,000. It was a composition generally of the type described in US Patent 4,655,767 (April 7, 1987).

### EXAMPLE 1 - Ascorbyl Phosphate

Following the mixing procedure detailed above in the specification, three compositions were prepared, and the contents and amounts of the ingredients used to form the compositions are shown in Table 1. In this example, the compositions contained varying amounts of the silicone pressure sensitive adhesive (PSA), the drug sodium ascorbyl phosphate (SAP), and the silicone polyether (SPE).

**Table 1 - Silicone Pressure Sensitive Adhesive/Drug/Silicone Polyether (percent by weight)**

| COMPOSITION | PSA | SAP | SPE | Observations |
|---|---|---|---|---|
| | | | | |
| 1 | 97 | 3 | 0 | Poor dispersion and large agglomerates |
| 2 | 94 | 3 | 3 | Uniform slurry, fine dispersion, good tack and adhesion |
| 3 | 91 | 3 | 6 | Uniform slurry, fine dispersion, good tack and adhesion |

In Table 1, Compositions 2 and 3 which are according to the invention, provided better dispersion properties than Composition 1 which was formulated without a silicone polyether.

### EXAMPLE 2 - Niacinamide

Example 1 was repeated and four more compositions were prepared containing a different drug. The contents and amounts of the ingredients used to form the compositions are shown in Table 2. In this example, the compositions contained varying amounts of the silicone pressure sensitive adhesive (PSA), the drug niacinamide (NIAC), and the silicone polyether (SPE).

**Table 2 - Silicone Pressure Sensitive Adhesive/Drug/Silicone Polyether (percent by weight)**

| COMPOSITION | PSA | NIAC | SPE | Observations |
|---|---|---|---|---|
| | | | | |
| 4 | 85 | 15 | 0 | Poor dispersion and large agglomerates |
| 5 | 90 | 5 | 5 | Good dispersion and no agglomerates |
| 6 | 80 | 10 | 10 | Good dispersion and no agglomerates |
| 7 | 70 | 15 | 15 | Good dispersion and no agglomerates |

In Table 2, Compositions 4-7 provided drug release rates of 46.5 percent, 64.9 percent, 51.4 percent, and 45.7 percent, respectively. This shows that Compositions 5 - 7, which are according to the present invention, each achieved significantly improved performance than Composition 4, which was formulated without a silicone polyether. Also, Compositions 5-7, which are according to the invention, provided better dispersion properties than Composition 4, which was formulated without a silicone polyether.

### EXAMPLE 3

Niacinamide and ketoconazole, the silicone polyether surfactant, and a solid excipient, were added to a solvated silicone pressure sensitive adhesive. The silicone polyether and the silicone pressure sensitive adhesive were the same compositions used in the previous examples. Laminates were prepared by using a table coater and some shims. The laminates were allowed to desolvate at ambient conditions. The laminates contained 90-100 percent by weight of the silicone pressure sensitive adhesive (PSA), 5 percent by weight of a silicone component which was either the silicone polyether (SPE) or a polydimethylsiloxane (PDMS) fluid having a viscosity of 10 centistoke (mm²/sec) used for comparison, and 5 percent by weight of the drugs Niacinamide or ketoconazole, based on the weight of the silicone matrix.

The drug dissolution was performed by means of Franz static diffusion cells. The drug analysis was performed by UV analysis. The physical properties of these compositions were evaluated by electron microscopy and dynamic rheological testing. The rheological testing protocol and the dynamic rheological testing equipment used herein are well known in the art, described in detail in the '767 patent, and reference may be had thereto. Thus, the rheological complex viscosity (Eta*), the elastic modulus (G'), and the viscous modulus (G") properties, were all evaluated and determined. It should be noted that dynamic rheological testing is a useful tool for evaluating the physicochemical properties of silicone matrices over time.

The rheolgical results according to this example are shown in Table 3. In the Table, values such as 3.0 E+06 mean 3.0 x 10⁶. Table 3 indicates that there is a strong interaction of the drug and the silicone polyether in the silicone matrix. These materials, when added to the silicone pressure sensitive adhesive, increase the Eta* (complex viscosity) and the G' (elastic modulus), which is beneficial. For example, the elastic modulus G' is a factor used in rheological profiles for calculating cross linking density. Another contributing factor to the high rheological values for these formulations is the presence in the silicone polyether of a hydrophobic moiety which is partitioned in the hydrophobic silicone matrix. Generally, therefore, the presence of the silicone polyether in the silicone matrix increases the cohesiveness of the matrix. This is beneficial as it permits drug formulators to add additional and other types of excipients such as permeation enhancers and drug release modulators, which excipients are known to decrease rheological properties and even contribute to cold flow resulting in oozing of adhesives.

**Table 3 - Silicone Matrix Containing Silicone Pressure Sensitive Adhesive, Silicone Polyether or Polydimethylsiloxane Fluid, and Drug (Percent by Weight).**

| Matrix | PSA | Drug | Silicone | Rheology Values @ 0.01 rad./sec. | | |
|---|---|---|---|---|---|---|
| | | | | Eta*(P) | G' (dyne/cm²) | G" (dyne/cm²) |
| | | | | | | |
| 1 | 100 % | 0 | 0 | 3.0 E+06 | 1.0 E+04 | 2.5 E+04 |
| 2 | 95 % | 5% Niacinamide | 0 | 8.5 E+07 | 4.4 E+05 | 7.3 E+05 |
| 3 | 90% | 5% Niacinamide | SPE | 7.4 E+08 | 5.0 E+06 | 4.9 E+06 |
| 4 | 95 % | 5% Ketoconazole | 0 | 7.0 E+06 | 4.2 E+04 | 5.6 E+04 |
| 5 | 90% | 5% Ketoconazole | SPE | 1.2 E+09 | 8.8 E+06 | 8.2 E+06 |
| 6 | 90% | 5% Ketoconazole | PDMS | 2.0 E+06 | 1.1 E+04 | 1.7 E+04 |
| 7 | 90% | 5% Niacinamide | PDMS | 1.6 E+07 | 9.0 E+04 | 1.3 E+05 |
| 8 | 90% | 5 % Ketoconazole | SPE | 6.5 E+08 | 4.3 E+06 | 4.9 E+06 |
| 9 | 90% | 5% Niacinamide | SPE | 2.9 E+08 | 1.8 E+06 | 2.3 E+06 |

These examples also demonstrate that the presence and use of a silicone polyether as a component of the silicone matrix provides several formulating advantages. Thus, (i) the silicone polyether functions as a carrier for incorporating solid drugs in a silicone matrix, (ii) acts as a crystal retardant for the drug and the excipient, and (iii) provides good in vitro flux rates and delivery profiles. As a processing aid, it enables the content uniformity compliance. Lastly, it is multi-functional to the extent that it compatiblizes solid drugs and hydrophilic excipient solids in the silicone matrix, thereby allowing the use of a hydrophobic silicone pressure sensitive adhesive for making transdermal patches containing hydrophilic solid drugs.

Other variations may be made in compounds, compositions, and methods described herein without departing from the essential features of the invention. The embodiments of the invention specifically illustrated herein are exemplary only and not intended as limitations on their scope except as defined in the appended claims.

## Claims

1. A method of making an adhesive matrix containing an adhesive and a solid powdered hydrophilic drug or a solid powdered hydrophilic excipient comprising the sequential steps of (i) forming a semi-solid composition containing the solid powdered hydrophilic drug or the solid powdered hydrophilic excipient, and a silicone polyether; (ii) adding to the semi-solid composition formed in (i) an adhesive or a solution containing a solvent and an adhesive; and (iii) mixing the semi-solid composition and the adhesive or the solution containing the solvent and the adhesive to form the adhesive matrix.

2. A method according to claim 1, herein the adhesive is hydrophobic.

3. A method according to Claim 1, wherein the adhesive is a silicone pressure sensitive adhesive.

4. A method according to Claim 1 further comprising (iv) applying the adhesive matrix to a substrate.

5. A method according to Claim 1 in which the solid powdered hydrophilic drug or the solid powdered hydrophilic excipient, and the silicone polyether, are present in the semi-solid composition in a weight ratio of 1:10 to 10:1.

6. A method according to Claim 1 in which the solution containing the adhesive and the solvent contains 10-90 percent by weight of the adhesive and 10-90 percent by weight of the solvent.

7. A method according to Claim 3 wherein the silicone pressure sensitive adhesive comprises (i) a silicone MQ resin containing monofunctional (M) units R₃SiO_{1/2} and tetrafunctional (Q) units SiO₄, wherein R is a hydrocarbon group; and (ii) a polydiorganosiloxane fluid or a polydiorganosiloxane gum.

8. A method according to claim 1 in which the silicone polyether is a copolymeric silicone polyether containing dimethylsiloxy repeating units and oxyalkylene functional siloxy repeating units, the copolymeric silicone polyether having a degree of polymerization less than about twenty.

9. An adhesive matrix comprising a solid powdered hydrophilic drug or a solid powdered hydrophilic excipient and a silicone polyether evenly dispersed in a silicone pressure sensitive adhesive, wherein the adhesive matrix is obtainable by the method of any one of the claims 1 to 8.

## Patentansprüche

1. Verfahren zur Herstellung einer Haftmittelmatrix, die ein Haftmittel und ein festes, pulverförmiges, hydrophiles Arzneimittel oder einen festen, pulverförmigen, hydrophilen Arzneimittelträger enthält, folgende aufeinander folgende Schritte umfassend: (I) Bilden einer halbfesten Zusammensetzung, die das feste, pulverförmige, hydrophile Arzneimittel oder den festen, pulverförmigen, hydrophilen Arzneimittelträger enthält, und eines Silikonpolyethers, (II) Zugeben eines Haftmittels oder einer Lösung, die ein Lösemittel und ein Haftmittel enthält, zu der in (I) gebildeten halbfesten Zusammensetzung und (III) Mischen der halbfesten Zusammensetzung und des Haftmittels oder der Lösung, die das Lösemittel und das Haftmittel enthält, um die Haftmittelmatrix zu bilden.

2. Verfahren nach Anspruch 1, wobei das Haftmittel hydrophob ist.

3. Verfahren nach Anspruch 1, wobei das Haftmittel ein druckempfindliches Silikonhaftmittel ist.

4. Verfahren nach Anspruch 1, ferner Folgendes umfassend: (IV) Aufbringen der Haftmittelmatrix auf ein Substrat.

5. Verfahren nach Anspruch 1, wobei das feste, pulverförmige, hydrophile Arzneimittel oder der feste, pulverförmige, hydrophile Arzneimittelträger und das Silikonpolyether in der halbfesten Zusammensetzung in einem Gewichtsverhältnis von 1:10 bis 10:1 vorhanden sind.

6. Verfahren nach Anspruch 1, wobei die Lösung, die das Haftmittel und das Lösemittel enthält, 10 bis 90 Gew.-% des Haftmittels und 10 bis 90 Gew.-% des Lösemittels enthält.

7. Verfahren nach Anspruch 3, wobei das druckempfindliche Silikonhaftmittel Folgendes umfasst: (I) ein Silikon-MQ-Harz, das monofunktionale (M) Einheiten R₃SiO_{1/2} und tetrafunktionale (Q) Einheiten SiO₄ enthält, wobei R eine Kohlenwasserstoffgruppe ist, und (II) ein Polydiorganosiloxan-Fluid oder einen Polydiorganosiloxan-Kautschuk.

8. Verfahren nach Anspruch 1, wobei das Silikonpolyether ein copolymeres Silikonpolyether ist, das Dimethylsiloxy-Wiederholungseinheiten und oxyalkylenfunktionale Siloxy-Wiederholungseinheiten enthält, wobei das copolymere Silikonpolyether einen Polymerisationsgrad von unter etwa 20 aufweist.

9. Haftmittelmatrix, ein festes, pulverförmiges, hydrophiles Arzneimittel oder einen festen, pulverförmigen, hydrophilen Arzneimittelträger und ein Silikonpolyether umfassend, die in einem druckempfindlichen Haftmittel gleichmäßig dispergiert sind, wobei die Haftmittelmatrix durch das Verfahren nach einem der Ansprüche 1 bis 8 gewonnen werden kann.

## Revendications

1. Procédé de production d'une matrice adhésive contenant un adhésif et un médicament hydrophile en poudre solide ou un excipient hydrophile en poudre solide comprenant les étapes séquentielles de (i) formation d'une composition semi-solide contenant le médicament hydrophile en poudre solide ou l'excipient hydrophile en poudre solide et un polyéther de silicone ; (ii) ajout dans la composition semi-solide formée en (i) d'un adhésif ou d'une solution contenant un solvant et un adhésif ; et (iii) mélange de la composition semi-solide et de l'adhésif ou de la solution contenant le solvant et l'adhésif pour former la matrice adhésive.

2. Procédé selon la revendication 1, dans lequel l'adhésif est hydrophobe.

3. Procédé selon la revendication 1, dans lequel l'adhésif est un adhésif silicone sensible à la pression.

4. Procédé selon la revendication 1, comprenant en outre (iv) l'application de la matrice adhésive sur un substrat.

5. Procédé selon la revendication 1, dans lequel le médicament hydrophile en poudre solide ou l'excipient hydrophile en poudre solide et le polyéther de silicone sont présents dans la composition semi-solide selon un rapport en poids de 1:10 à 10:1.

6. Procédé selon la revendication 1, dans lequel la solution contenant l'adhésif et le solvant contient 10 à 90 pour cent en poids de l'adhésif et 10 à 90 pour cent en poids du solvant.

7. Procédé selon la revendication 3, dans lequel l'adhésif silicone sensible à la pression comprend (i) une résine MQ de silicone contenant des motifs monofonctionnels (M) R₃SiO_{1/2} et des motifs tétrafonctionnels (Q) SiO₄, où R est un groupe hydrocarboné ; et (ii) un fluide de polydiorganosiloxane ou une gomme de polydiorganosiloxane.

8. Procédé selon la revendication 1, dans lequel le polyéther de silicone est un polyéther de silicone copolymère contenant des motifs diméthylsiloxy répétés et des motifs oxyalkylènesiloxy fonctionnels répétés, le polyéther de silicone copolymère ayant un degré de polymérisation inférieur à vingt environ.

9. Matrice adhésive comprenant un médicament hydrophile en poudre solide ou un excipient hydrophile en poudre solide et un polyéther de silicone dispersés de façon homogène dans un adhésif silicone sensible à la pression, laquelle matrice adhésive peut être obtenue par le procédé selon l'une quelconque des revendications 1 à 8.
